**Europäisches Patentamt**

**European Patent Office**

**Office européen des brevets**

(11) Publication number: **0 466 367 B1**

# EUROPEAN PATENT SPECIFICATION

(45) Date of publication of patent specification: **23.08.95**  (51) Int. Cl.6: **C12Q 1/68**

(21) Application number: **91305837.6**

(22) Date of filing: **27.06.91**

(54) **Process for detecting nucleic acid.**

(30) Priority: **28.06.90 JP 170684/90**

(43) Date of publication of application:
**15.01.92 Bulletin 92/03**

(45) Publication of the grant of the patent:
**23.08.95 Bulletin 95/34**

(84) Designated Contracting States:
**CH DE FR GB IT LI NL**

(56) References cited:
**EP-A- 0 235 726**
**EP-A- 0 407 789**
**WO-A-87/05334**
**GB-A- 2 156 074**

(73) Proprietor: **WAKUNAGA SEIYAKU KABUSHIKI KAISHA**
**5-36, Miyahara 4-chome**
**Yodogawa-ku**
**Osaka-Shi**
**Osaka 532 (JP)**

(72) Inventor: **Yamane, Akio, c/o Wakunaga Seiyaku K.K.**
**1624, Shimo Koutachi,**
**Kouda-Cho**
**Takata-Gun,**
**Hiroshima-Ken (JP)**

(74) Representative: **Dowden, Marina et al**
**Elkington and Fife**
**Prospect House,**
**8 Pembroke Road**
**Sevenoaks,**
**Kent TN13 1XR (GB)**

Rank Xerox (UK) Business Services
(3.10/3.09/3.3.3)

**Description**

BACKGROUND OF THE INVENTION

Field of the Invention

The present invention relates to a process for detecting a nucleic acid. More particularly, it is concerned with a process for detecting a target nucleic acid which enables a target nucleic acid to be rapidly detected and the detection procedure to be automated, which process comprises immobilizing single stranded nucleic acids which have a sequence that is complementary to a target nucleic acid sequence to be detected on a solid support and hybridizing the single stranded nucleic acid with a labelled target nucleic acid contained in a sample.

Description of the Related Art

Basic hybridization processes for detecting a specific sequence of a nucleic acid include a solid-liquid hybridization process wherein nucleic acids adsorbed on a solid support are hybridized with nucleic acids contained in a solution and a liquid-liquid hybridization process wherein target nucleic acids and nucleic acid probes contained in a solution are hybridized with each other (Anal. Biochem., 169, 1-25 (1988)). Up to now, these processes have been improved in various ways for the purpose of more easily, more rapidly and more sensitively detecting a target nucleic acid (Anal. Biochem., 169, 1-25 (1988)).

Among them, there is a proposal for a process wherein a microtiter well used in the field of immunoassay is utilized for automating the procedure of detecting a target nucleic acid (Japanese Patent Laid-Open Publication No. 219400/86). In this process, double stranded DNA in a sample are converted to a single stranded form, and the single stranded nucleic acids are immobilized through a nonspecific adsorption on a microtiter well under such a condition that complementary strands are present. Due to the inherent characteristics of DNA, some of the single stranded nucleic acids return to a double stranded form during the immobilization process and the subsequent hybridization process, so that in an actual hybridization, the amount of the hybridizable DNA appears to be substantially smaller than that of immobilized DNA. Further, in the immobilization process, the amount of DNA immobilized is relatively small which renders this process unsatisfactory from the practical viewpoint.

Immobilization of polythymidylic acids on a microtiter well has been proposed as a capturing probe for a sandwich hybridization process (Molecular and Cellular Probes, 3, 189-207 (1989)). This process is advantageous in that the amount of DNA immobilized is much larger than that in the case of a mixed base sequence of DNA by virtue of immobilization of a polymer of thymidylic acid having a higher reactivity than that of other nucleic acid bases in a photoreaction. The application of immobilized polythymidylic acids, however, is limited to a sandwich hybridization, with the disadvantages of complicated procedure and lower sensitivity inherent in the sandwich hybridization.

GB 2156074 further describes an improved sandwich hybridization process utilising nucleic acid reagents which comprise an array of nucleic acid fragments. The use of such arrays of nucleic acid fragments in the nucleic acid reagents are particularly well suited for use in the more complex sandwich hybridization process.

A nucleic acid amplification process called PCR (Polymerase Chain Reaction) was developed in recent years. It is the epoch-making process which can amplify a minute amount of a certain gene by one hundred thousand times or more in a short time (U.S. Patent No. 4,683,195). A proposal has been made for a process for detecting a mutation of human gene through the utilization of nucleic acids amplified by PCR (Proc. Natl. Acad. Sci., USA, 86, 6230-6234 (1889)). The proposed process is called "reverse dot hybridization" and characterized by chemically synthesed oligonucleotides complementary to a base sequence to be detected and adding polythymidylic acid to the oligonucleotides by an enzymatic reaction to facilitate the immobilization of the oligonucleotide on a nylon membrane. This process has the advantage of oligonucleotide being efficiently immobilized on a nylon membrane, but is disadvantageous in that the process wherein use is made of a nylon membrane, as such, is unsuitable for automation of the detection procedure and the preparation of the immobilized probes are unsuitable for mass production.

Finally, EP 0235726 describes a method of detecting one or more microorganisms or polynucleotide sequences from eukaryotic sources in a nucleic acid-containing test sample which involves immobilizing a single-stranded nucleic acid of one or more known microorganisms or sequences from eukaryotic sources.

Although the above processes have advantages in some means, they have respective drawbacks and no process capable of realizing a satisfactory simpleness, rapidity and high sensitivity has been developed in the art.

## SUMMARY OF THE INVENTION

The present inventors have found that a target nucleic acid can be simply and rapidly detected with high sensitivity through the use of a solid support wherein single stranded nucleic acids containing between 5 to 200 repeat sequences which are complementary to a target nucleic acid and which are immobilized to place the single stranded nucleic acid sequences in such a state that a strand complementary to the nucleic acid is absent, which has led to the completion of the present invention.

Accordingly, an object of the present invention is to provide a process capable of detecting a target nucleic acid in a simple, rapid and highly sensitive manner.

Another object of the present invention is to provide a process for detecting a target nucleic acid which can easily by automated.

The process for detecting a nucleic acid comprises the following steps:

(i) labelling a target nucleic acid to be detected;

(ii) immobilizing single stranded nucleic acids which are specifically hybridizable with the target nucleic acid, on a solid support to place said single stranded nucleic acid under such a condition that a strand complementary to said single stranded nucleic acid is absent;

(iii) hybridizing said target nucleic acid labelled in step (i) with said single stranded nucleic acid immobilized in step (ii) and washing said solid support; and

(iv) detecting, simultaneously with or after step (iii), said target nucleic acid through the utilization of a label present in said target nucleic acid, characterised in that said single stranded nucleic acid is derived from either a phage or a composite vector comprising a phage and a plasmid and said single stranded nucleic acid further has a plurality of sequences hybridizable with said target nucleic acid, said sequences being repeated 5 to 200 times.

The process for detecting a nucleic acid according to the present invention enables a target nucleic acid to be specifically detected with high sensitivity and high efficiency and further enables the detection procedure to be improved in the simpleness and rapidity and to be automated.

## BRIEF DESCRIPTION OF THE DRAWINGS

The aforesaid and other objects and features of the present invention will now become apparent from the following detailed description with reference to the accompanying drawings, in which:

Fig. 1 is a flow diagram showing the construction of an improved vector for preparing a linear single stranded DNA and the preparation of the single stranded DNA;

Fig. 2 is a graph showing the results of a comparison of the hybridization efficiency on a single stranded DNA immobilized on a microtiter well with that on a double stranded DNA immobilized on a microtiter well;

Fig. 3 is a diagram showing nucleic acid sequences inserted into a vector for the preparation of an immobilized single stranded DNA in the detection of point mutation of $\beta$-thalassemia gene;

Fig. 4 is a flow diagram showing the preparation of a plasmid vector containing repetition of a oligonucleotide unit complementary to a human papilloma virus 16 gene;

Fig. 5 is a graph showing the relationship between the number of oligonucleotide units of an immobilized probe and the sensitivity; and

Fig. 6 is a diagram showing sequences of respective types of HLA-DB genes and a sequence of a probe used in the present invention.

## DESCRIPTION OF THE PREFERRED EMBODIMENTS

## Target Nucleic Acid

The term "target nucleic acid to be detected" used in the present invention is intended to mean a nucleic acid containing a specific base sequence to be detected and may be any of DNA and RNA. The nucleic acids to which the present invention is applicable can be prepared from every form of live including bacteria, virus and higher plants and animals. Further, when the the detection process according to the present invention is applied, the nucleic acids may be in a purified form or in an unpurified form.

In the nucleic acid detection process according to the present invention, not only a nucleic acid obtained from the above-described form of life but also a synthesized nucleic acid corresponding to a nucleic acid derived from the specimen or a synthesized nucleic acid complementary to the nucleic acid may be used as the target nucleic acid to be detected. Therefore, in the present invention, the "target nucleic acid" includes besides a specimen-derived nucleic acid to be detected, a synthesized nucleic acid corresponding to the specimen-derived nucleic acid to be detected and further a synthesized nucleic acid complementary to the nucleic acid.

Nucleic Acid Detection Process

Step (i): Step of labelling target nucleic acid

In the nucleic acid detection process according to the present invention, a target nucleic acid to be detected is first labelled. Examples of the labelling method include (1) one wherein a label is directly introduced into a target nucleic acid, (2) one wherein a nucleic acid corresponding to the target nucleic acid or a nucleic acid complementary to the target nucleic acid is synthesized through the use of a labelled oligonucleotide primer and (3) one wherein a nucleic acid corresponding to the target nucleic acid or a nucleic acid complementary to the target nucleic acid is synthesized through the use of an oligonucleotide primer in the presence of a labelled nucleotide unit.

A method wherein a biotin derivative is introduced into a target nucleic acid by a photoreaction and a detection is conducted by the use of enzyme conjugated streptoavidin (Nucleic Acids Res., 13, 745 (1985)) and a method wherein a target nucleic acid is sulfonated and detected by the use of an enzyme conjugated anti-sulfon antibody (Proc. Natl. Acad. Sci., USA, 81, 3466-3470 (1984)) are preferred as the method (1) wherein a label is directly introduced into a target nucleic acid from the viewpoints of simpleness and rapidity of the procedure.

An amplification of a specific nucleic acid sequence (BIO/TECHNOLOGY, 8, 291 (1990)) may be utilized for the methods (2) and (3). The methods have drawn attention particularly in respect of the amplification of a target nucleic acid and further are of a high value in relatively simple labelling of a synthesized nucleic acid corresponding to a target nucleic acid or a synthesized nucleic acid complementary to a target nucleic acid. For example, in the PCR process (Science, 230, 1350-1354 (1985)), a labelled elongation product or amplification product can be prepared through the utilization of a labelled primer or labelled mononucleotides. In the amplification method wherein use is made of $Q\beta$ replicase (BIO/TECHNOLOGY, 6, 1197 (1988)), a labelled elongation product or amplification product can be prepared through the utilization of similarly labelled mononucleotides. Also in the nucleic acid amplification method other than described above, an elongation product or an amplification product can be labelled with labelled mononucleotides or labelled oligonucleotide incorporated by an elongation reaction or an amplification reaction. The method (2) is preferred in the present invention.

The label used herein may be radioactive or non-radioactive as far as this substance can be detected after the hybridization procedure. A non-radioactive label is preferred from the viewpoint of handleability, storage stability and disposal and because it can exhibit most efficiently the effect of the present invention.

Examples of the non-radioactive label include haptens, such as biotin, 2,4-dinitrophenyl group and digoxigenin, fluorescent substances such as fluorescein, rhodamine, tetramethylrhodamine, sulfo rhodamine, 7-nitrobenz-2-oxa-1,3-diazole (NBD) and dansyl group or chemiluminescent substances such as acridine. The oligonucleotide can be labelled with the substance by any of the known means (Japanese Patent Laid-Open Publications No. 93098/84 and 93099/84). When labelled nucleotides are used, the labelling can be conducted by known means (Proc. Natl. Acad. Sci., USA, 80, 4045 (1983) and Japanese Patent Laid-Open Publication No. 152364/88). Alternatively, a commercially available product may be utilized.

Step (ii): Immobilization of single stranded nucleic acid

In the present step, single stranded nucleic acids specifically hybridizable with a target nucleic acid are immobilized on a solid support to place the immobilized single stranded nucleic acid under such a condition that a strand complementary to the single stranded nucleic acid is absent.

In the present invention, when the single stranded nucleic acid is DNA, it may be one prepared, for example, by denaturing a double stranded nucleic acid and subsequent strand separation (Nucleic Acids Res., 13, 5457-5468 (1985)). Further, it may be one prepared by synthesizing a single stranded nucleic acid through the use of a DNA polymerase and separating the synthesized single stranded nucleic acid from a template (Anal. Biochem., 162, 130-136 (1987)). Further, it is also possible to utilize a single stranded nucleic acid prepared from an M13 phage containing a gene to be detected, or a single stranded nucleic acid prepared from a composite vector comprising a phage and a plasmid (for example, pUC118, pBSM13+ and PUCf1) (Methods in Enzymology, 153, 3-34 (1987)). When the single stranded nucleic acid is RNA, it may be not only a naturally occurring RNA but also RNA synthesized in vitro through the utilization of an RNA polymerase or the like. If a base sequence other than a sequence specifically hybridizable with a target nucleic acid contained in the nucleic acid is inconvenient for the hybridization reaction, the inconvenient portion can be removed according to the Messing's method (Methods in Enzymology, 101, part C, 20 (1983)).

According to another aspect of the present invention, the single stranded nucleic acid to be immobilized is preferably one containing a plurality of sequences hybridizable with a target nucleic acid. This is because the presence of many sequences hybridizable with a target nucleic acid in a single stranded nucleic acid to be immobilized can shorten the time necessary for the hybridization, so that a gene can be rapidly detected.

The single stranded nucleic acid containing a plurality of sequences hybridizable with a target nucleic acid include one prepared by introducing a plurality of sequences hybridizable with a target nucleic acid into the above-described phage DNA or a composite vector comprising a phage and a plasmid and preparing a single stranded nucleic acid therefrom. In particular, a single stranded nucleic acid prepared from a vector containing 5 to 200 copies of a sequence is used which is hybridizable with a target nucleic acid.

When a point mutation or the like in a nucleic acid sequence in a sample is detected by hybridization, it is preferred that the sequence hybridizable with the target nucleic acid is relatively short.

In general, single stranded nucleic acids are immobilized on a solid support by the following method. In some cases, however, the immobilization efficiency is poor depending upon the immobilization method. Even in such a case, the immobilization through the use of single stranded nucleic acids containing a plurality of sequences hybridizable with a target nucleic acid is advantageous in that the sequence hybridizable with a target nucleic acid can be immobilized in a relatively large amount.

In general, it is known that the immobilization of the oligonucleotide comprising a sequence hybridizable with a target nucleic acid on a solid support causes the portion participating in the immobilization on the support to lose its degree of freedom and consequently to become impossible for it to participate in the hybridization. Thus, the hybridization efficiency of the immobilized oligonucleotides is lower than that attained in the solution. When use is made of single stranded nucleic acids prepared through the use of the above-described vector containing a plural number of a unit sequence hybridizable with a target nucleic acid for the preparation of single strand DNA, however, a number of the unit sequence not involved in the immobilization on a solid support appear to be present. Thus, the lowering in the hybridization efficiency derived from the immobilization is advantageously small.

However, when the labelling is conducted through the use of an elongation reaction or an amplification reaction and the labelled product to be detected, the single stranded nucleic acid to be immobilized is preferably less homologous to primers utilized for the elongation reaction or amplification reaction. For example, in the PCR process, in many cases, the primer used in the gene amplification remains in the solution after the amplification reaction. In this case, it is necessary to select the sequence of the single stranded nucleic acid in such a manner that the primers do not hybridize with the single stranded nucleic acid. This is true of the other gene amplification methods.

The support for immobilizing the single stranded nucleic acid may be any material or any shape as long as a nucleic acid can be nonspecifically adsorbed, or a functional group can be introduced and a covalent bond can be formed between the functional group and the nucleic acid. Specific examples of the carrier include polymer microtiter well, tube and bead. The use of the microtiter well is particularly preferred from the viewpoint of ease of automation of the procedure.

One of the methods of immobilizing single stranded nucleic acids on a solid support is a chemical binding method (e.g. Nucleic Acids Res., 15, 5373-5390 (1987)). Examples of the method of immobilizing the nucleic acid through a chemical bond include one wherein a carrier containing an amino group introduced thereinto is bound to a nucleic acid through the use of a crosslinking agent such as glutaraldehyde. Further, the introduction of a functional group (for example, a primary amino group through a transamination reaction) into a nucleic acid followed by binding of the introduced functional group onto a solid support through the use of a suitable crosslinking agent is also useful.

It is also possible to directly immobilize a nucleic acid onto a solid support through a nonspecific binding such as adsorption. In particular, when the support is a microtiter well, the adsorption efficiency can be enhanced through an ultraviolet irradiation or the addition of $MgCl_2$ (Japanese Patent Laid-Open Publication No. 219400/86). A further useful method is that in which a nucleic acid and a protein are chemically conjugated or nonspecifically adsorbed to each other and the immobilization is conducted by taking advantage of a nonspecific adsorption of the protein to a carrier.

Thus, the immobilization of the fully hybridizable single stranded nucleic acids on a solid support can be achieved.

Step (iii): Hybridization step

In this step, the target nucleic acid labelled in step (i) is hybridized with the single stranded nucleic acid immobilized in step (ii).

The hybridization conditions may be properly selected and determined according to a combination of the target nucleic acid with the immobilized single stranded nucleic acid. For example, the hybridization in the present step can be basically conducted in the same manner as that wherein use is made of the conventional membranes (B.D. Hames and S.J. Higgins, Nucleic Acid Hybridization, A Practical Approach, IRL Press (1985)).

In some solid supports, the nonspecific adsorption to the nucleic acid becomes weak depending upon what hybridization conditions are used. In such a case, the procedure of the prehybridization reaction can be omitted. For the same reason, the composition of the solution can be simplified. When the hybridization reaction is conducted for a long period of time, the immobilized single stranded nucleic acids may often be liberated. Thus, it is preferred to conduct the hybridization under such conditions that the hybridization time can be reduced.

The washing procedure after the hybridization reaction as well can be conducted in the same manner as that of the conventional process. Because of simplicity, it is preferred to conduct the washing procedure under such conditions that excess reagents etc. can be removed at room temperature.

In the detection of a point mutation, the washing conditions should be carefully determined. It is also useful to utilize such conditions wherein the stability of duplex depends upon only the length of the complementary strand but does not depend upon the base composition of the complementary strand (Nucleic Acids Res., 16, 4637-4650 (1988)).

Step (iv): Step of detection

In this step, simultaneously with or after step (iii), a target nucleic acid is detected through the utilization of a label present in the target nucleic acid.

The detection procedure in the present step may be properly selected and determined according to the kind of the label present in the target nucleic acid.

When the label present in the target nucleic acid is directly detectable, that is, when the label is, for example, a radioisotope, a fluorescent substance or a dye, the detection procedure may be conducted in such a state that a labelled nucleic acid is bonded to a solid phase, or alternatively the detection procedure may be conducted by liberating the label into a solution in such a state that it is bonded to the nucleic acid or released from the nucleic acid and then conducting the detection according to the label. On the other hand, when the label is indirectly detectable, that is, when the label is a ligand capable of causing a specific binding reaction, such as biotin or hapten, the detection procedure can be conducted by a method commonly used in the detection of this type, that is, through the use of an acceptor (for example, avidin or antibody) to which a label capable of directly generating a signal or an enzyme catalyzing a signal generating reaction has been bound. The acceptor may be previously added in step (iii). In this case, the binding process of the ligand to the acceptor can be conducted simultaneously with step (iii) which results in the whole process being simplified.

EXAMPLES

The present invention will now be described in more detail by way of the following examples, though it is not limited to these examples only.

The procedure of the genetic engineering techniques in the following examples was conducted according to Molecular Cloning, the 2nd edition (T. Maniatis et al., Cold Spring Harbar Laboratory Press (1989)). Oligonucleotide was prepared through the use of a model 381 A automatic synthesizer manufactured by Applied Biosystems, Inc. and subjected to deprotection reaction and purification before use by the conventional procedure (Oligonucleotide Synthesis, IRL Press (1984)). The biotin-labelled oligonucleotide was prepared by adding Aminolink II (trademark) (manufactured by Applied Biosystems, Inc.) to oligonucleotide in the final stage of the synthesis of the oligonucleotide to introduce an amino group into the oligonucleotide, and reacting the oligonucleotide with biotin succinimide ester according to the method described in U.S. Patent No. 4,849,336.

Reference Example 1

Preparation of single stranded DNA for immobilization

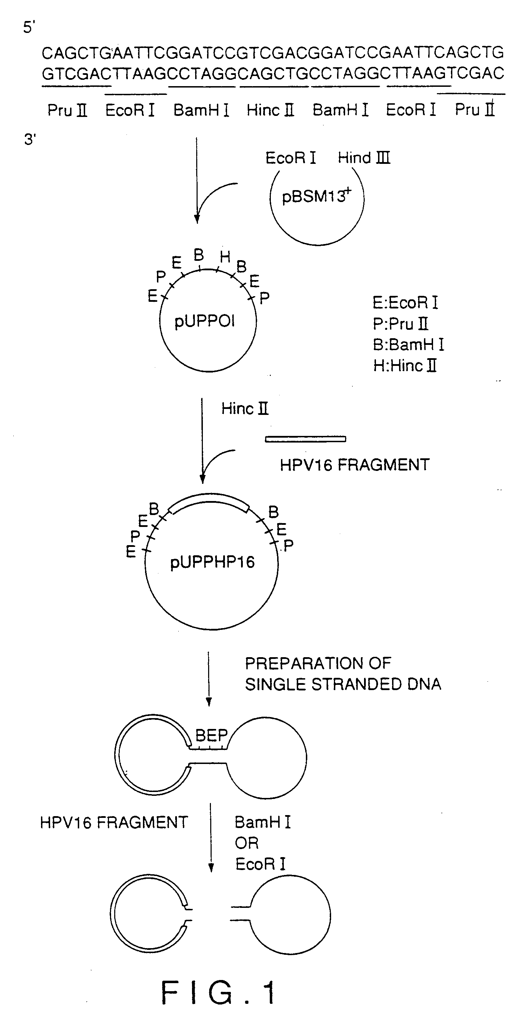

In order to prepare a linear single stranded DNA, use was made of a modification of a method by Messing et al. (Methods in Enzymology, 101, Part C, 20 (1983)). A chemically synthesized DNA fragment shown in Fig. 1 was inserted in between EcoRI cleaved site and Hind III cleaved site of plasmid pBSM13+ (manufactured by Stratagene Cloning Systems, Inc.) to prepare plasmid pUPPO1. Then, a 1.8 kb fragment containing genes E6 and E7 of human papilloma virus 16 was inserted into a Hinc II cleaved site of this plasmid to prepare plasmid pUPPHP16. E. coli NM522 was transformed with the plasmid, and a single stranded DNA was prepared by the conventional procedure through the use of helper phage M13K07 (Methods in Enzymology, 153, 3-34 (1987)).

The single stranded DNA was cleaved by restriction enzyme EcoRI or BamHI for linearization.

Reference Example 2

Immobilization of single stranded DNA on microtiter well

The single stranded DNA prepared in Reference Example 1 was dissolved in a solution of 10 mM Tris•HCl, pH 7.6, and 1 mM EDTA to a concentration of 100 ng/$\mu$l and then mixed with a four-fold volume of H$_2$O and a five-fold volume of an immobilization buffer (1.5 M NaCl, 0.3 M Tris•HCl, pH 8.0, 0.3 M MgCl$_2$). The mixture was added to a microtiter well (Dynatech, Immulon 2, removawell strips, No. 011-010-6302) in an amount of 100 $\mu$l per well. The well was covered and allowed to stand at 37°C for 16 hr. Then, the liquid was removed, and the well was air-dried at 37°C for 30 min and subjected to light irradiation at a dose of 500,000 $\mu$J through the use of Stratalinker (trademark) 2400 (manufactured by Stratagene Cloning Systems, Inc.). After the light irradiation, the well was washed three times with a washing buffer (1 M NaCl, 2 mM MgCl$_2$, 0.1 M Tris•HCl, pH 9.3, 0.1% Tween 20 registered trade mark : 200 $\mu$l). The well was then sealed in a polyvinyl chloride bag and stored at 4°C.

Reference Example 3

Hybridization in microtiter well and detection

A hybridization solution (5 x SSC, 5 x Denhardt's solution, 0.2% SDS, 200 $\mu$g/ml, salmon sperm DNA: 100 $\mu$l/well) was added to the well on which the single stranded DNA containing a human papilloma virus 16 gene prepared in Reference Example 2 had been immobilized, and further a serial dilution of biotin-labelled oligonucleotides (Bio-ATTGTAATGGGCTCTGTCCG, 20 ng/well) complementary to part of the human papilloma virus 16 gene was added thereto. The mixture was maintained at 55°C for 30 min. The hybridization solution was removed, and the well was washed three times with 2 x SSC (200 $\mu$l/well). A streptoavidin-alkaline phosphatase solution (prepared by subjecting a solution of streptavidin-alkaline phosphatase (Bethesda Research Laboratories, Inc.) to 1,000-fold dilution with 0.1 M Tris•HCl, pH 7.5, 0.3 M NaCl, 2 mM MgCl$_2$, 0.05% (v/v) Triton X-100) was added thereto (100 $\mu$l/well), and the well was shaken at 23°C for 10 min. The reaction mixture was removed from the well, and the well was washed three times with a washing solution (0.1 M Tris•HCl, pH 7.5, 0.3 M NaCl, 2 mM MgCl$_2$, 0.05% (v/v) Triton X-100 registered trade mark : 200 $\mu$l/well). After the washing, a p-nitrophenyl phosphate solution (1 M diethanolamine, pH 9.8, 0.5 mM MgCl$_2$: 4 mg/ml: 100 $\mu$l/well) was added to the well and a reaction was allowed to proceed at 23°C for one hr. Absorbance was then measured at 405 nm. The results are given in Table 1.

Table 1

|  | Absorbance (at 405 nm) |
|---|---|
| Plate on which single stranded DNA containing a human papilloma virus sequence has been immobilized | 1.34 |
| Plate on which single stranded DNA unrelating a human papilloma virus sequence has been immobilized | 0.13 |
| Plate on which no DNA has been immobilized | 0.13 |

The numerical value in Table 1 is one determined from the absorbance at 405 nm by subtracting the background value derived from the substrate itself.

Reference Example 4

Effect of UV irradiation on immobilization of DNA on microtiter well

The plasmid DNA (pUPPHP16) prepared as in Reference Example 1 and single stranded DNA prepared therefrom were linearized by cleaving with EcoRI and immobilized on a plate in the same manner as that of Reference Example 2. In this case, when double stranded DNA was immobilized, heat denaturation was conducted before the double stranded DNA was mixed with the immobilization buffer. Then, two types of plates were prepared. Specifically, in one plate, UV irradiation was conducted in the same manner as that of Reference Example 2, and in another plate, no UV irradiation was conducted. The hybridization capability of the plates thus prepared was examined through the use of Bio-ATTGTAATGGGCTCTGTCCG in the same manner as that of Reference Example 3. The results are given in Table 2.

Table 2

|  | UV irradiation | No UV irradiation |
|---|---|---|
| Double stranded DNA | 0.51 | 0.48 |
| Single stranded DNA | 1.58 | 0.58 |

The numerical value in Table 2 was determined from the absorbance at 405 nm by subtracting the background value derived from the substrate itself.

Reference Example 5

Comparison of hybridization capability after immobilization on plate of single stranded DNA with that of double stranded DNA

The hybridization capability after immobilization on a plate of single stranded DNA was compared with the hybridization capability after immobilization on a plate of denatured double stranded DNA. The single stranded DNA and the double stranded DNA were prepared in substantially the same manner as that of Reference Example 4. The single stranded DNA were immobilized in a cyclic form.

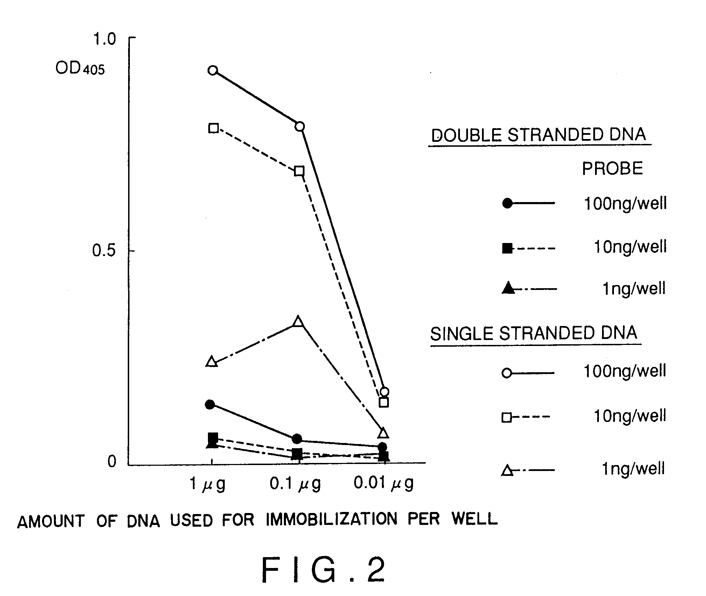

DNA was added in an amount of 1 $\mu$g, 100 ng and 10 ng each per well for immobilization in the same manner as that of Reference Example 2. The hybridization capability of each plate was examined through the use of Bio-ATTGTAATGGGCTCTGTCCG as a probe in the same manner as that of Reference Example 3. The results were as shown in Fig. 2 (the absorbance at 405 nm was measured by a microplate reader).

Reference Example 6

Labelling by gene amplification and detection of amplification product and detection of point mutation

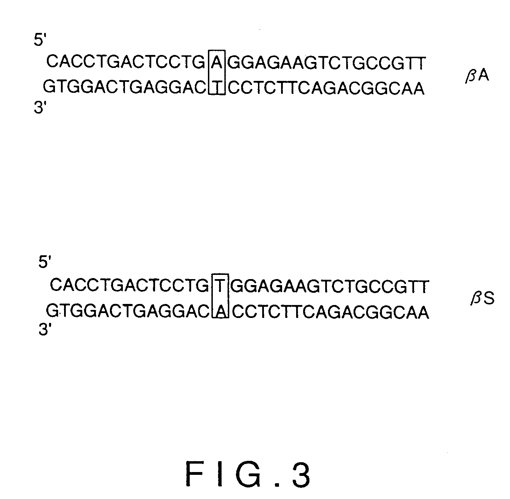

In order to detect a human $\beta$-globin gene, an oligonucleotide corresponding to the 2nd to 11th codons was chemically synthesized and inserted into a HincII site of plasmid pUCfl (see Fig. 3). The procedure was conducted for both a normal gene (βA) and a point mutated gene (βS) causative of β-thalassemia. Among the resultant clones, a clone capable of providing a sense single stranded DNA was selected, and single stranded DNA was prepared therefrom. The single stranded DNA thus prepared were immobilized in a cyclic form on a plate according to the procedure described in Reference Example 2.

Then, a gene amplification reaction was conducted. The reaction was conducted through the use of GeneAmp (trademark) manufactured by Cetus Corporation according to the protocol. 5′ ACACAACTGTGTGTTCACTAGC and biotinylated Bio-CAACTTCATCCACGTTCACC were used as the primer, and 4.4 kb DNA fragments prepared from the plasmid DNA (pBR322-HβPst) by the digestion of Pst I (DNA, 3, 7-15 (1984)) was used as the template.

After the gene amplification, an aliquot of the reaction mixture was heat-denatured and subjected to hybridization and subsequent detection in the same manner as that of Reference Example 3. The results are given in Table 3 (the measurements are those determined by a microplate reader).

### Table 3

| | Absorbance (at 405 nm) |
|---|---|
| Plate on which DNA containing βA sequence has been immobilized | 0.432 |
| Plate on which DNA containing βS sequence has been immobilized | 0.288 |
| Plate on which DNA unrelating to β-globin has been immobilized | 0.037 |

Example 7

Preparation of single stranded DNA containing repetition of unit sequence

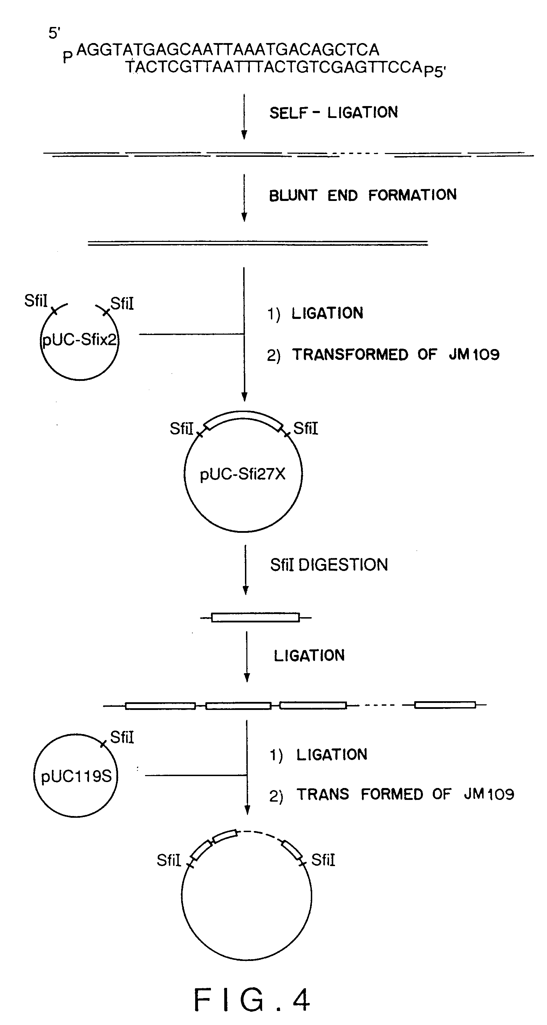

A part of E7 gene of human papilloma virus 16 was chemically synthesized, and single stranded DNA containing repetition of a sequence of the synthesized oligonucleotides (the unit sequence) was prepared therefrom according to the method shown in Fig. 4.

At the outset, two kinds of oligonucleotides shown in Fig. 4 were chemically synthesized, and the 5′ terminal thereof was phosphorylated with polynucleotide kinase and ATP. Then, the two kinds of oligonucleotides were mixed with each other to form a double strand. The fragments of the double strand were linked to each other through the use of T4 DNA ligase. After ligation, blunt ends were formed through the use of Klenow fragment of E. coli DNA polymerase and four kinds of deoxynucleoside triphosphates. The resultant reaction product was electrophoresed on 6% polyacrylamide gel, and a portion corresponding to 270 bp was cut out to recover DNA.

Then, plasmid pUC-Sfix2 (Japanese Patent Laid-Open Publication No. 190194/90) was cleaved by a restriction enzyme BamHI, and the terminal 5′ phosphate was removed with alkaline phosphatase. This product was ligated with T4 DNA ligase to the DNA recovered from the polyacrylamide gel. E. coli JM109 was transformed with the ligated product. An intended clone was selected for resistibility of ampicillin. The plasmid pUC-Sfi27x thus obtained was cleaved by restriction enzyme SfiI, and a portion containing repetition of the unit sequence was purified and recovered through the use of electrophoresis. This fragment was self-ligated with T4 DNA ligase and inserted into a restriction enzyme SfiI site of plasmid pUC119S (prepared by inserting SfiI linker (manufactured by Boehringer Mannheim; #909785) into a BamHI cleaved site of pUC119). Clones are different from each other in the number of the unit sequence depending upon the degree of self-ligation. In the present experiment, clones wherein the numbers of the unit sequence is 9, 14, 64 and 165 were obtained.

Example 8

Effect of probe into which oligonucleotide has been repeatedly inserted

Single stranded DNAs were prepared respectively from the clone prepared in Example 7 and a separately prepared clone having one unit sequence in the same manner as that of Reference Example 1 and immobilized on a microtiter well in the same manner as that of Reference Example 2.

Then, the following gene amplification was conducted through the use of the following two biotin-labelled primers:

Bio-GCAACCAGAGACAACTGATC

Bio-ATTGTAATGGGCTCTGTCCG

and a plasmid containing a E7 gene of papilloma virus 16 (HPV16) as a template.

GeneAmp (trademark) reagent manufactured by Cetus Corporation was added to 10 ng of plasmid pUPPHP16 (see Reference Example 1) and 100 ng of each primer according to the protocol, and the total volume of the reaction solution was adjusted to 100 $\mu$l with water. The reaction solution was heated at 95°C for 5 min in a thermal cycler manufactured by Perkin-Elmer Cetus Instruments, Inc. to denature DNA, and 2.5 units of AmpliTaq (trademark) was added thereto. The cycle of heating at 72°C for 60 sec, at 94°C for 30 sec and at 50°C for 30 sec was repeated 30 times.

The reaction mixture was diluted with water and 5 $\mu$l of the diluent was heat-denatured and hybridized with a previously prepared microtiter well on which the single stranded DNA containing a E7 gene sequences has been immobilized under the same conditions as those described in Reference Example 3, and the absorbance was measured.

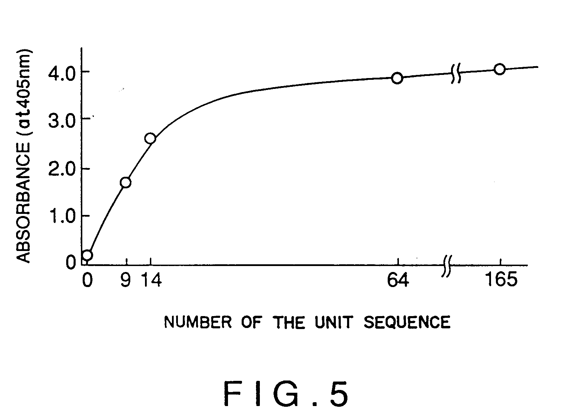

The results are shown in Fig. 5. As is apparent from the drawing, when the number of the unit sequence was 64 or 165, the sensitivity was about 20 times that in the case where the number of the unit sequence was one.

Example 9

Detection of human papilloma virus 16 gene in rapid and simple manner

In order to simplify the hybridization process of the present invention further, studies were conducted on the composition of the hybridization solution, hybridization temperature and color development time, and the detection of a human papilloma virus 16 gene was conducted under the following simplified conditions.

10 ng of DNA extracted from Caski cells as a positive sample and 10 ng of DNA extracted from human peripheral blood as a negative sample were each used as a template, and gene amplification was conducted by the PCR process in the same manner as that of Example 8. The resultant reaction mixtures were added to wells prepared as in Example 8. On one well single stranded DNA wherein the number of repetitions of the unit sequence was one had been immobilized, and on the other well, single stranded DNA wherein the number of repetitions of the unit sequence was 64 had been immobilized. Hybridization was conducted in 100 $\mu$l of a hybridization solution (5 x SSC, 0.2% SDS) with 5 $\mu$l of the above-described PCR mixture at 37°C for 30 min. The hybridization solution was then removed from the well, and the well was washed three times with 200 $\mu$l of a washing solution ( 2 x SSC).

A streptoavidin-alkaline phosphatase solution (prepared by subjecting a solution of streptoavidinalkaline phosphatase (manufactured by Bethesda Research Laboratories, Inc.) to 1,000-fold dilution with 0.1 M Tris-HCl, pH 7.5, 0.3 M NaCl, 2 mM MgCl$_2$, 0.05% (v/v) Triton X-100) was added thereto 100 $\mu$l/well, and the plate was gently shaken at 23°C for 10 min. The solution was removed from the well, and the well was washed three times with a washing solution (0.1 M Tris.HCl, pH 7.5, 0.3 M NaCl, 2 mM MgCl$_2$, 0.05% (v/v) Triton X-100 (registered trade mark) : 200 $\mu$l/well). Alter the washing, 100 $\mu$l of p-nitrophenyl phosphate solution (1 M diethanolamine, pH 9.8, 0.5 mM MgCl$_2$: 4 mg/ml) was added to the well and an enzymatic reaction was allowed to proceed at 23°C for 20 min. The absorbance was then measured at 405 nm. The results are given in Table 4. As is apparent from Table 4, even when the procedure was simplified, the positive sample could be significantly distinguished from the negative sample in the case of single stranded DNA wherein the number of the unit sequence was 64.

Table 4

| Plate sample | Number of the unit sequence is 1 | Number of the unit sequence is 64 |
|---|---|---|
| Positive sample (derived from Caski cells) | 0.04 | 0.73 |
| Negative sample (derived from normal human) | 0.00 | 0.02 |

Note: * The numerical value in Table 4 is one determined from the absorbance at 405 nm by subtracting the background value derived from the substrate itself.

Example 10

Detection of mutation of HLA-DRB gene

Gene amplification was conducted through the use of the following two kinds of biotin-labelled primers [GLPDRBI: corresponding to the 17th to 23rd amino acids of HLA-DRB, GAMPDRBI: corresponding to the 87th to 94th amino acids of HLA-DRB, J. Exp. Med., 169, 2263-2267 (1989)]:

Bio-TTCTTCAATGGGACGGAGCG

Bio-GCCGCTGCACTGTGAAGCTCTC

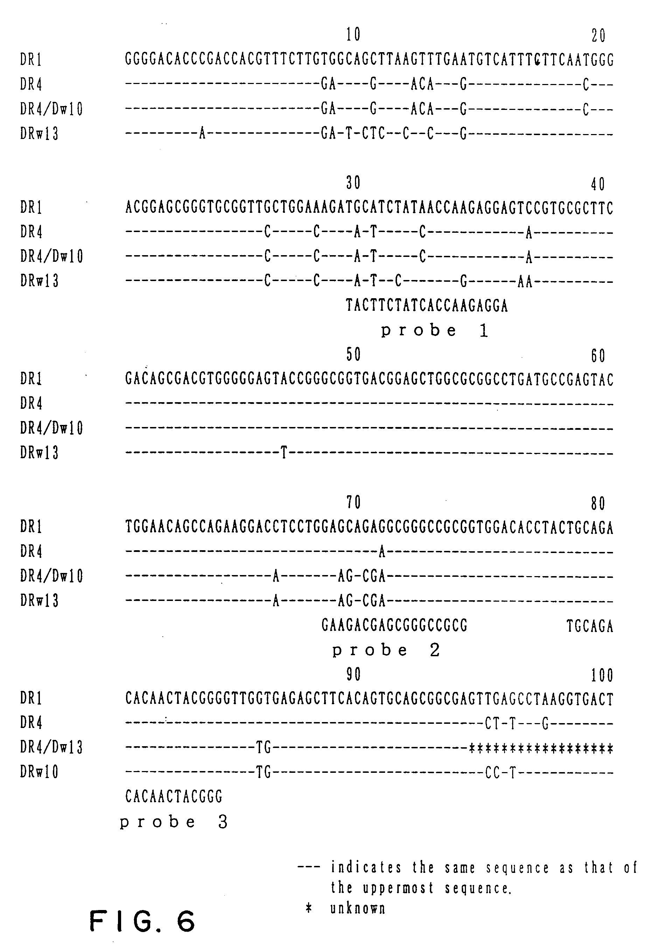

and 1 μg of DNA extracted from human peripheral blood as a template in the same manner as that of Example 8. Unit sequences as shown in Fig. 6 (probe 1, 2, and 3) were repeatedly ligated in the same manner as that of Example 7 and immobilized on a well, respectively. Probe 1 is completely matched to a gene sequence of genotype DR4, while probe 2 is completely matched to a gene sequence of genotypes DR4/DW 10 and DRW 13 (see Fig. 6). Probe 3 is complementary to a common sequence of all the genotypes. In probes 1 and 2, the number of the unit sequence was 50, while in probe 3, the number of the unit sequence was 10.

The gene amplification reaction cycle of heating at 94°C for 30 sec, at 50°C for 30 sec and at 72°C for 60 sec was repeated 30 times. 5 μl of the reaction mixture was added to wells on which probes 1, probe 2 and probe 3 had been immobilized, and the hybridization was conducted at 60°C for one hr and the enzymatic reaction was conducted at 23°C for one hr. Other detailed procedure was as same as that of Reference Example 3.

The results are given in Table 5. Both wells on which probe 1 and probe 2 were immobilized exhibited a high absorbance specifically with genotypes expecting to have a high homology in the sequence. These results demonstrate that the present invention is useful for the typing of the HLA gene.

Table 5

| Sample Genotype | Probe 1 | Probe 2 | Probe 3 |
|---|---|---|---|
| 1 DR4/DW 15, DR9 | 7.5* | 0.12 | 10.1* |
| 2 DR4/DW 15 | 13.7* | 0.18 | 9.0* |
| 3 DRW 13, DRW 14 | 0.35 | 6.6* | 17.8* |
| 4 DR9, DRW 13 | 1.5 | 1.5* | 10.0* |
| 5 DRW 8/DW 8.3, DR9 | 6.9 | 0.17 | 12.1* |
| 6 DRW 14 | 0.37 | 0.19 | 18.0* |
| 7 DR2 | 0.67 | 0.10 | 15.3* |

Note:

* Asterisk (*) in Table 5 is estimated that the probe is completely matched to the gene sequence of the genotype.
** The numerical value in Table 5 is one determined from the absorbance at 405 nm by subjecting the background value derived from the subsequent itself. When the absorbances were beyond two, the value obtained here was converted from that of a diluted solution.

**Claims**

1. A process for detecting a nucleic acid comprising the steps of:
   (i) labelling a target nucleic acid to be detected;
   (ii) immobilizing single stranded nucleic acids which are specifically hybridizable with the target nucleic acid, on a solid support to place said single stranded nucleic acid under such a condition that a strand complementary to said single stranded nucleic acid is absent;
   (iii) hybridizing said target nucleic acid labelled in step (i) with said single stranded nucleic acid immobilized in step (ii) and washing said solid support; and
   (iv) detecting, simultaneously with or after step (iii), said target nucleic acid through the utilization of a label present in said target nucleic acid, characterised in that said single stranded nucleic acid is derived from either a phage or a composite vector comprising a phage and a plasmid and said single stranded nucleic acid further has a plurality of sequences hybridizable with said target nucleic acid, said sequences being repeated 5 to 200 times.

2. A method of detecting a nucleic acid according to claim 1, wherein said target nucleic acid labelled in step (i) is a synthesized nucleic acid corresponding to the nucleic acid derived from a specimen or a synthesized nucleic acid complementary to the nucleic acid derived from a specimen.

3. A method of detecting a nucleic acid according to claim 1 or 2, wherein said single stranded nucleic acid immobilized in step (ii) contains a plurality of sequences specifically hybridizable with the target nucleic acid.

4. A method of detecting a nucleic acid according to claim 1 or 2, wherein said single stranded nucleic acid immobilized in step (ii) contains 5 to 200 sequences specifically hybridizable with the target nucleic acid.

**Patentansprüche**

1. Verfahren zum Nachweis einer Nukleinsäure, umfassend die Stufen:
   (i) Markieren einer nachzuweisenden Zielnukleinsäure,
   (ii) Immobilisieren von einzelsträngigen Nukleinsäuren, die mit der Zielnukleinsäure spezifisch hybridisierbar sind, auf einem festen Träger, um die einzelsträngige Nukleinsäure in einen solchen Zustand zu bringen, daß ein, der einzelsträngigen Nukleinsäure komplementärer, Strang fehlt,

(iii) Hybridisieren der in Stufe (i) markierten Zielnukleinsäure mit der in Stufe (ii) immobilisierten einzelsträngigen Nukleinsäure und Waschen des festen Trägers, und
(iv) gleichzeitig mit oder nach Stufe (iii) Nachweisen der Zielnukleinsäure über die Verwendung eines in der Zielnukleinsäure vorhandenen Markers, **dadurch gekennzeichnet**, daß die einzelsträngige Nukleinsäure entweder von einem Phagen oder einem zusammengesetzten Vektor, umfassend einen Phagen und ein Plasmid, abgeleitet ist und die einzelsträngige Nukleinsäure weiterhin eine Vielzahl von mit der Zielnukleinsäure hybridisierbaren Sequenzen besitzt, wobei die Sequenzen 5 bis 200fach wiederholt sind.

2. Verfahren zum Nachweis einer Nukleinsäure nach Anspruch 1, wobei die in Stufe (i) markierte Zielnukleinsäure eine entsprechend der von einer Probe abgeleiteten Nukleinsäure synthetisierte Nukleinsäure ist oder eine der von einer Probe abgeleiteten Nukleinsäure komplementäre synthetisierte Nukleinsäure ist.

3. Verfahren zum Nachweis einer Nukleinsäure nach Anspruch 1 oder 2, wobei die in Stufe (ii) immobilisierte einzelsträngige Nukleinsäure eine Vielzahl von spezifisch mit der Zielnukleinsäure hybridisierbaren Sequenzen enthält.

4. Verfahren zum Nachweis einer Nukleinsäure nach Anspruch 1 oder 2, wobei die in Stufe (ii) immobilisierte einzelsträngige Nukleinsäure 5 bis 200 spezifisch mit der Zielnukleinsäure hybridisierbare Sequenzen enthält.

**Revendications**

1. Procédé pour détecter un acide nucléique, qui comprend les étapes consistant :
(i) à marquer un acide nucléique cible à détecter ;
(ii) à immobiliser des acides nucléiques simple brin, qui peuvent subir une hybridation spécifique avec l'acide nucléique cible, sur un support solide, pour placer ledit acide nucléique simple brin dans un état tel que soit absent un brin complémentaire dudit acide nucléique simple brin ;
(iii) à hybrider ledit acide nucléique cible, marqué dans l'étape (i), avec ledit acide nucléique simple brin immobilisé dans l'étape (ii), et à laver ledit support solide ; et
(iv) à détecter, en même temps que l'étape (iii) ou après cette dernière, ledit acide nucléique cible par utilisation d'un marqueur présent dans ledit acide nucléique cible, caractérisé en ce que ledit acide nucléique simple brin dérive d'un phage ou d'un vecteur composite comprenant un phage et d'un plasmide, et en ce que ledit acide nucléique simple brin possède, en outre, une pluralité de séquences pouvant être hybridées, avec ledit acide nucléique cible, lesdites séquences étant répétées 5 à 200 fois.

2. Procédé pour détecter un acide nucléique selon la revendication 1, dans lequel l'acide nucléique cible, marqué dans l'étape (i), est un acide nucléique synthétisé, correspondant à l'acide nucléique qui dérive d'un spécimen ou d'un acide nucléique synthétisé complémentaire de l'acide nucléique obtenu à partir d'un spécimen.

3. Procédé pour détecter un acide nucléique selon la revendication 1 ou 2, dans lequel ledit acide nucléique simple brin, immobilisé dans l'étape (ii), contient une pluralité de séquences pouvant subir une hybridation spécifique, avec l'acide nucléique cible.

4. Procédé pour détecter un acide nucléique selon la revendication 1 ou 2, dans lequel ledit acide nucléique simple brin, immobilisé dans l'étape (ii), contient de 5 à 200 séquences pouvant subir une hybridation spécifique, avec l'acide nucléique cible.

5'

CAGCTGAATTCGGATCCGTCGACGGATCCGAATTCAGCTG
GTCGACTTAAGCCTAGGCAGCTGCCTAGGCTTAAGTCGAC

PruⅡ  EcoRI  BamHI  HincⅡ  BamHI  EcoRI  PruⅡ

3'

EcoRI    HindⅢ

pBSM13⁺

pUPPOI

E:EcoRI
P:PruⅡ
B:BamHI
H:HincⅡ

HincⅡ

HPV16 FRAGMENT

pUPPHP16

PREPARATION OF
SINGLE STRANDED DNA

BEP

HPV16 FRAGMENT    BamHI
OR
EcoRI

# F I G . 1

AMOUNT OF DNA USED FOR IMMOBILIZATION PER WELL

# F I G . 2

EP 0 466 367 B1

5'
CACCTGACTCCTG[A]GGAGAAGTCTGCCGTT
GTGGACTGAGGAC[T]CCTCTTCAGACGGCAA    βA
3'

5'
CACCTGACTCCTG[T]GGAGAAGTCTGCCGTT
GTGGACTGAGGAC[A]CCTCTTCAGACGGCAA    βS
3'

# FIG.3

5'
P AGGTATGAGCAATTAAATGACAGCTCA
TACTCGTTAATTTACTGTCGAGTTCCA P5'

↓ SELF – LIGATION

↓ BLUNT END FORMATION

SfiI    SfiI
pUC-Sfix2 ——— 1) LIGATION

2) TRANSFORMED OF JM109

SfiI    SfiI
pUC-Sfi27X

↓ SfiI DIGESTION

↓ LIGATION

SfiI
pUC119S ——— 1) LIGATION

2) TRANS FORMED OF JM109

SfiI    SfiI

# F I G . 4

FIG.5

```
                                        10                          20
DR1       GGGGACACCCGACCACGTTTCTTGTGGCAGCTTAAGTTTGAATGTCATTTGTTCAATGGG
DR4       -----------------------GA----G----ACA---G-------------C---
DR4/Dw10  -----------------------GA----G----ACA---G-------------C---
DRw13     ---------A-------------GA-T-CTC--C--C---G-----------------

                                        30                          40
DR1       ACGGAGCGGGTGCGGTTGCTGGAAAGATGCATCTATAACCAAGAGGAGTCCGTGCGCTTC
DR4       ------------------C-----C----A-T-----C-----------A----------
DR4/Dw10  ------------------C-----C----A-T-----C------------A----------
DRw13     ------------------C-----C---A-T--C-------G------AA----------
                                              TACTTCTATCACCAAGAGGA
                                                 p r o b e   1
                                        50                          60
DR1       GACAGCGACGTGGGGGAGTACCGGGCGGTGACGGAGCTGGCGCGGCCTGATGCCGAGTAC
DR4       ------------------------------------------------------------
DR4/Dw10  ------------------------------------------------------------
DRw13     -----------------T------------------------------------------

                                        70                          80
DR1       TGGAACAGCCAGAAGGACCTCCTGGAGCAGAGGCGGGCCGCGGTGGACACCTACTGCAGA
DR4       --------------------------------A---------------------------
DR4/Dw10  -----------------A-------AG-CGA-----------------------------
DRw13     -----------------A-------AG-CGA-----------------------------
                                       GAAGACGAGCGGGCCGCG          TGCAGA
                                            p r o b e   2
                                        90                          100
DR1       CACAACTACGGGGTTGGTGAGAGCTTCACAGTGCAGCGGCGAGTTGAGCCTAAGGTGACT
DR4       --------------------------------------------CT-T---G--------
DR4/Dw13  ----------------TG--------------------------***************
DRw10     ----------------TG-------------------------CC-T-----------
          CACAACTACGGG
          p r o b e   3
```

--- indicates the same sequence as that of
the uppermost sequence.
*  unknown

# FIG. 6